(19) Europäisches Patentamt
European Patent Office
Office européen des brevets

(11) EP 3 636 686 A1

(12) EUROPEAN PATENT APPLICATION

(43) Date of publication:
15.04.2020 Bulletin 2020/16

(21) Application number: 18199701.6

(22) Date of filing: 10.10.2018

(51) Int Cl.:
*C08G 18/73 (2006.01)* *A61Q 5/06 (2006.01)*
*C08G 18/75 (2006.01)* *C08G 18/08 (2006.01)*
*C08G 18/42 (2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Covestro Deutschland AG**
**51373 Leverkusen (DE)**

(72) Inventor: **The designation of the inventor has not yet been filed**

(74) Representative: **Levpat**
**c/o Covestro AG**
**Gebäude 4825**
**51365 Leverkusen (DE)**

# (54) COSMETIC FORMULATIONS OR COMPOSITIONS COMPRISING BIODEGRADABLE FILM FORMERS

(57) A film forming polyurethane dispersion is described comprising a biodegradable polyurethane which is built from at least the following components:
a) at least one aliphatic, araliphatic and/or cycloaliphatic polyisocyanate, with an NCO functionality in a range of from 1.8 to 4, preferably of from 2 to 3.
b) at least one polyol having a number-average molecular weight Mn of ≥ 330 and ≤ 6000 g/mol, or preferably of ≥ 350 and ≤ 5000 g/mol and a hydroxyl functionality of ≥ 1.5 and ≤ 4,
c) optionally at least one amino-functional compound which has at least two isocyanate-reactive amino groups,
d) optionally at least one alcohol which has at least two hydroxyl groups and a molar mass of ≥ 62 and ≤ 399 g/mol,
e) optionally at least one compound which has one group that is reactive towards isocyanate groups,
wherein the polyurethane provides a biodegradation rate according to OECD Guideline 301 B in water of at least 50 % in 28 days. Also the use of the film forming polyurethane dispersion to increase the biodegradation rate of a cosmetic is describes as well as a method to use the polyurethane dispersion for shaping hair styles is described.

Figure 1

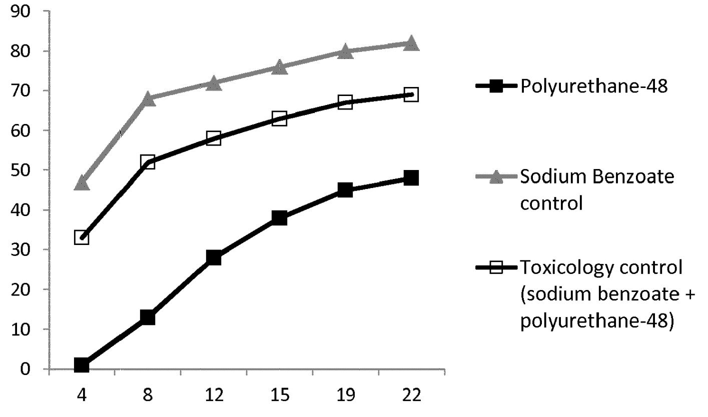


EP 3 636 686 A1

## Description

The present invention relates to a film forming polyurethane dispersion comprising a biodegradable polyurethane, to a cosmetic formulation comprising the film forming polyurethane as well as the use of the film forming polyurethane dispersion in cosmetic formulations.

The biodegradability of artificially produced products is one of the future problems to be solved. To keep rivers and the sea clean from micro plastics the need for biodegradable polymers is high.

Therefore, it was an aim of the present invention, to provide film formers which might be used in cosmetic formulations, also called composition, which provide a biodegradability which is acceptable for the environment.

A first aspect of the invention is a film forming polyurethane dispersion comprising a biodegradable polyurethane which is built from at least the following components:

a) at least one aliphatic, araliphatic and/or cycloaliphatic diisocyanate,
b) at least one polyol having a number-average molecular weight Mn of ≥ 330 and ≤ 6000 g/mol, or preferably of ≥ 350 and ≤ 5000 g/mol and a hydroxyl functionality of ≥ 1.5 and ≤ 4,
c) optionally at least one amino-functional compound which has at least two isocyanate-reactive amino groups,
d) optionally at least one alcohol which has at least two hydroxyl groups and a molar mass of ≥ 60 and ≤ 399 g/mol, preferably in an amount of ≤ 10 wt.-%, or preferably in an amount of ≤ 5 wt.-%, based on the total weight of component b), component d) and any further alcohol or polyol used,
e) optionally at least one compound which has one group that is reactive towards isocyanate groups,

wherein the polyurethane provides a biodegradation rate according to OECD Guideline 301 B in water of at least 50 % in 28 days.

In the context of the invention, polyurethanes are polymeric compounds which have at least two, preferably at least three, urethane-group-containing repeat units

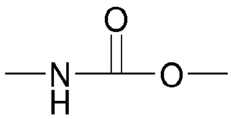

The polyurethanes preferably comprise one or more urea-group-containing repeat units:

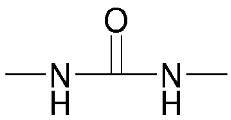

In the context of the invention, when polyurethanes are described they also cover polyurethanes which contain polyurea groups, hereinafter referred to as polyurethaneurea. Therefore, in those cases where only polyurethaneureas are mentioned also polyurethanes are meant.

Preferably, the polyurethane or polyurethaneurea used according to the invention is composed of

a) at least one aliphatic, araliphatic and/or cycloaliphatic polyisocyanate, with an NCO functionality in a range of from 1.8 to 4, preferably of from 2 to 3, preferably at least one aliphatic diisocyanate,
b) at least one polyol with a number-average molecular weight Mn ≥ 400 and ≤ 6000 g/mol and a hydroxyl functionality of ≥ 1.5 and ≤ 4, preferably of ≥ 1.6 and ≤ 3, or preferably of ≥ 1.8 and ≤ 2.3,
c) optionally at least one amino functional compound which has at least two isocyanate-reactive amino groups,
d) optionally at least one alcohol which has at least two hydroxyl groups and a molar mass ≥ 62 and ≤ 399 g/mol, preferably ≥ 70 and ≤ 300 g/mol,
e) optionally at least one compound which has one group that is reactive towards isocyanate groups and
f) optionally ≤ 20 wt.-%, based on the total mass of the polyurethane, of at least one polyol with a number-average molecular weight Mn ≥ 500 and ≤ 6000 g/mol and a hydroxyl functionality of ≥ 1.5 and ≤ 4, which is different from b),
g) optionally a hydrophilizing component.

In the context of this application, the number-average molecular weight is always determined by gel permeation chromatography (GPC) in tetrahydrofuran at 23°C. The procedure here is in accordance with DIN 55672-1: "Gel permeation chromatography, Part 1 - Tetrahydrofuran as eluent" (SECurity GPC System from PSS Polymer Service, flow

rate 1.0 ml/min; columns: 2×PSS SDV linear M, 8×300 mm, 5 μm; RID detector). Here, polystyrene samples of known molar mass are used for the calibration. The calculation of the number-average molecular weight is assisted by software. Baseline points and evaluation limits are stipulated according to DIN 55672 Part 1.

Furthermore, the polyurethane or polyurethaneurea is preferably composed of ≥ 5 and ≤ 60 wt.-% of component a), ≥ 30 and ≤ 90 wt.-% of component b), ≥ 2 and ≤ 25 wt.-% of component c), ≥ 0 and ≤ 10 wt.-% of component d), ≥ 0 and ≤ 10 wt.-% of component e) and ≥ 0 and ≤ 20 wt.-% of component f), and ≥ 0 and ≤ 10 wt.-% of component g), preferably ≥ 0.1 and ≤ 15 wt.-% in each case based on the total mass of the polyurethane, where the components a) to g) add up to less or equal to 100 wt.-%.

Furthermore, the polyurethane is preferably composed of ≥ 10 and ≤ 40 wt.-% of component a), ≥ 55 and ≤ 85 wt.-% of component b), ≥ 5 and ≤ 20 wt.-% of component c), ≥ 0 and ≤ 3 wt.-% of component d), ≥ 0 and ≤ 3 wt.-% of component e) and ≥ 0 and ≤ 1 wt.-% of component f), and ≥ 0 and ≤ 20 wt.-% of component g), preferably ≥ 0.1 and ≤ 15 wt.-% of component g) in each case based on the total mass of the polyurethane, where the components a) to g) add up to less or equal to 100 wt.-%.

Compounds suitable as component a) are for example 1,4 butylene diisocyanate, 1,5 pentamethylene diisocyanate (PDI), 1,6 hexamethylene diisocyanate (HDI), isophorone diisocyanate (IPDI), 2,2,4 and/or 2,4,4 trimethylhexamethylene diisocyanate, the isomeric bis(4,4' isocyanate-cyclohexyl)methanes or mixtures thereof with any desired isomer content (H12-MDI), 1,4 cyclohexylene diisocyanate, 4 isocyanatomethyl-1,8-octane diisocyanate (nonane triisocyanate), 1,3 and/or 1,4 bis(2-isocyanatoprop-2-yl)benzene (TMXDI), 1,3 bis(isocyanatomethyl)benzene (XDI), and alkyl 2,6 diisocyanatohexanoates (lysine diisocyanates) with C1-C8-alkyl groups.

Besides the polyisocyanates specified above, modified diisocyanates or triisocyanates with isocyanurate, urethane, allophanate, biuret, iminooxadiazinedione and/or oxadiazinetrione structure can also be co-used proportionately. Preferably, the amount of modified diisocyanates or triisocyanates is ≤ 10 wt.-%, or preferably ≤ 5 wt.-%, based on the total weight of component a). The polyisocyanates of component a) are preferably polyisocyanates or polyisocyanate mixtures of the type specified above with exclusively aliphatically and/or cycloaliphatically bonded isocyanate groups and an average NCO functionality of the mixture of 2 to 4, preferably of 2 to 2.6 and particularly preferably of 2 to 2.4. Furthermore, preferably, no aromatic polyisocyanates are used for producing the polyurethane or polyurethaneurea.

Preferably, the component a) is selected from IPDI, HDI and/or H12-MDI or mixtures of at least two thereof. Particularly preferably, the component a) is HDI. Preferably, the polyisocyanate component a) comprises ≥ 80 mol%, preferably ≥ 85 mol%, further preferably 95 mol% and particularly preferably 100 mol%, of an aliphatic diisocyanate, preferably of IPDI, HDI, H12-MDI or a mixture of at least two thereof.

Preferably, the component a) comprises diisocyanates, where the diisocyanates are selected from aliphatic, araliphatic and/or cycloaliphatic diisocyanates. Preferably, the diisocyanates have at least one isocyanate group which is bonded to a secondary and/or tertiary carbon atom.

Polyols suitable as component b) are polyesterpolyols, polyacrylatepolyols, polyurethanepolyols, polycarbonatepolyols, polyesterpolyacrylatepolyols, polyurethanepolyacrylatepolyols, polyurethanepolyesterpolyols, polyurethanepolyetherpolyols, polyurethanepolycarbonatepolyols, polyetherpolycarbonatepolyols and/or polyesterpolycarbonatepolyols, in particular polyesterpolyols and/or polycarbonatepolyols, known per se in polyurethane technology, especially in the polyurethane coating technology. These polyols can be used in component b) individually or in any desired mixtures of at least two thereof.

It is likewise preferred if the polyol component b) has a number-average molecular weights of ≥ 330 and ≤ 6000 g/mol, or preferably from 350 to 5000 g/mol and/or an average OH functionalities of 1.5 to 6, preferably from 1.8 to 3 and particularly preferably from 1.9 to 2.1.

It is likewise advantageous if the polyol component b) comprises a polyester, preferably a polyester based on adipic acid, or consists thereof.

The expression "polymeric" polyols, like polyester polyol or polyether polyol, means in particular that the specified polyols have at least two, more preferably at least three, repeat units joined together.

Polyesterpolyols are for example the polycondensates, known per se, of di- and optionally tri-, and tetraols and di- and optionally tri- and tetracarboxylic acids or hydroxycarboxylic acids or lactones. Instead of the free polycarboxylic acids, the corresponding polycarboxylic anhydrides or corresponding polycarboxylic acid esters of lower alcohols can also be used for preparing the polyesters.

Examples of diols suitable for the preparation of polyesterpolyols are ethylene glycol, butylene glycol, diethylene glycol, triethylene glycol, polyalkylene glycols, such as polyethylene glycol, also 1,2-propanediol, 1,3-propanediol, butanediol(1,3), butanediol(1,4), hexanediol(1,6) and isomers, neopentyl glycol or neopentyl glycol hydroxypivalate, with hexanediol(1,6) and isomers, neopentyl glycol and neopentyl glycol hydroxypivalate being preferred. In addition, polyols such as trimethylolpropane, glycerol, erythritol, pentaerythritol, trimethylolbenzene or trishydroxyethyl isocyanurate can also be used.

Dicarboxylic acids which can be used are phthalic acid, isophthalic acid, terephthalic acid, tetrahydrophthalic acid, hexahydrophthalic acid, cyclohexanedicarboxylic acid, adipic acid, azelaic acid, sebacic acid, glutaric acid, tetrachlorophthalic acid, maleic acid, fumaric acid, itaconic acid, malonic acid, suberic acid, 2-methylsuccinic acid, 3,3-diethylglutaric acid and/or 2,2-dimethylsuccinic acid. The corresponding anhydrides may also be used as acid source.

If the average functionality of the polyol to be esterified is greater than 2, it is also possible to additionally co-use monocarboxylic acids, such as benzoic acid and hexanecarboxylic acid.

Preferred acids are aliphatic or aromatic acids of the aforementioned type. Particular preference is given to adipic acid, isophthalic acid and optionally trimellitic acid, very particularly preferably adipic acid.

Hydroxycarboxylic acids which can be co-used as reactants in the production of a polyester polyol with terminal hydroxyl groups are for example hydroxycaproic acid, hydroxybutyric acid, hydroxy-decanoic acid, hydroxystearic acid and the like. Suitable lactones are caprolactone, butyrolactone and homologs. Preference is given to caprolactone.

Preferred components b) for the preparation of the polyurethanes are polyester polyols with a number-average molecular weight of from 330 to 3000 g/mol, in particular aliphatic polyester polyols based on aliphatic carboxylic acids and aliphatic polyols, in particular based on adipic acid and aliphatic alcohols, such as hexanediol and/or neopentyl glycol.

In component b), it is also possible to use polycarbonates having hydroxyl groups, preferably polycarbonate diols, having number-average molecular weights Mn of 400 to 8000 g/mol, preferably from 600 to 3000 g/mol. These are obtainable by reaction of carbonic acid derivatives, such as diphenyl carbonate, dimethyl carbonate or phosgene, with polyols, preferably diols. Preferably, component b) comprises polycarbonate polyols in an amount in a range of from 0 to 5 wt.-%, or preferably in a range of from 0.1 to 2 wt.-%, based on the total weight of component b).

Examples of such diols are ethylene glycol, 1,2- and 1,3-propanediol, 1,3- and 1,4-butanediol, 1,6-hexanediol, 1,8-octanediol, neopentyl glycol, 1,4-bishydroxymethylcyclohexane, 2-methyl-1,3-propanediol, 2,2,4-trimethylpentanediol-1,3, dipropylene glycol, polypropylene glycols, dibutylene glycol, polybutylene glycols, bisphenol A and lactone-modified diols of the aforementioned type. The polycarbonates having hydroxyl groups are preferably linear in structure. Polyether polyols can likewise be used in component b). Of suitability are for example the polytetramethylene glycol polyethers known per se in polyurethane chemistry, as are obtainable by polymerization of tetrahydrofuran by means of cationic ring opening.

Likewise suitable polyether polyols are the addition products, known per se, of styrene oxide, ethylene oxide, propylene oxide, butylene oxides and/or epichlorohydrin onto di- or polyfunctional starter molecules.

Suitable starter molecules that can be used are all compounds known according to the prior art, such as, for example, water, butyl diglycol, glycerol, diethylene glycol, trimethylolpropane, propylene glycol, sorbitol, ethylenediamine, triethanolamine, 1,4-butanediol. Preferred starter molecules are water, ethylene glycol, propylene glycol, 1,4-butanediol, diethylene glycol and butyl diglycol.

Preferably, component b) comprises polyether polyols in an amount in a range of from 0 to 5 wt.-%, or preferably in a range of from 0.1 to 2 wt.-%, based on the total weight of component b).

Preferably, it is provided that as component c) an amino-functional compound which has at least two isocyanate-reactive amino groups, also called chain extender, is used. Preferably, the component c) is selected from ethylendiamine, isophoron diamine (IPDA) or a mixture of these. Preferably, the component c) comprises ≥ 85 mol%, preferably ≥ 95 mol% and particularly preferably 100 mol%, 1,2-ethylenediamine or isophorone diamin (IPDA) or a mixture of these.

As further constituents of the component c), further $NH_2$- and/or NH-functional compounds can be used besides IPDA.

Preferably, the chain extension/termination is carried out before dispersion in water, the isocyanate groups reacting with the chain extender to give urea groups.

Suitable components - which are preferably used in addition to IPDA in a molar fraction of less than 25% - are di- or polyamines such as 1,2- and 1,3-diaminopropane, 1,4-diaminobutane, 1,6-diaminohexane, 2,2,4- and 2,4,4-trimethylhexamethylenediamine, 2-methylpentamethylenediamine, diethylenetriamine, triaminononane, 1,3- and 1,4-xylylenediamine, $\alpha,\alpha,\alpha',\alpha'$-tetramethyl-1,3- and -1,4-xylylenediamine and 4,4-diaminodicyclohexylmethane and/or dimethylethylenediamine. Hydrazine or and hydrazides such as adipic dihydrazide are likewise possible.

Apart from components a) and b) and optionally c), further building blocks can also be used for producing the polyurethanes or polyurethane urea according to the invention.

Examples are hydroxy-functional compounds of component d) with molecular weights of 62 to 399 g/mol, such as, for example, polyols of the specific molecular weight range having up to 20 carbon atoms, such as ethylene glycol, diethylene glycol, triethylene glycol, 1,2-propanediol, 1,3-propanediol, 1,4-butanediol, 1,3-butylene glycol, cyclohexanediol, 1,4-cyclohexanedimethanol, 1,6-hexanediol, neopentyl glycol, hydroquinone dihydroxyethyl ether, bisphenol A (2,2-bis(4-hydroxyphenyl)propane), hydrogenated bisphenol A, (2,2-bis(4-hydroxycyclohexyl)propane), trimethylolpropane, glycerol, pentaerythritol. Furthermore, monofunctional isocyanate-reactive amine compounds can be used, such as, for example, methylamine, ethylamine, propylamine, butylamine, octylamine, laurylamine, stearylamine, isononyloxypropylamine, dimethylamine, diethylamine, dipropylamine, dibutylamine, n-methylaminopropylamine, diethyl(methyl)-aminopropylamine, morpholine, piperidine.

In addition, for example as component e), it is also possible to use monofunctional isocyanate-reactive hydroxyl-group-containing compounds to prepare the polyurethane according to the invention. Examples of such monofunctional

compounds are ethanol, n-butanol, ethylene glycol monobutyl ether, diethylene glycol monomethyl ether, diethylene glycol monobutyl ether, propylene glycol monomethyl ether, dipropylene glycol monomethyl ether, tripropylene glycol monomethyl ether, dipropylene glycol monopropyl ether, propylene glycol monobutyl ether, dipropylene glycol - monobutyl ether, tripropylene glycol monobutyl ether, 2-ethylhexanol, 1-octanol, 1-dodecanol, 1-hexadecanol or mixtures of at least two thereof.

In one preferred embodiment of the invention, the polyurethane used according to the invention comprises $\leq$ 10 wt.-%, preferably $\leq$ 5 wt.-%, or preferably 0.1 to 5 wt.-% of component e), in each case based on the total mass of the polyurethane, yet more preferably component e) is not used for the preparation of the polyurethane.

Further examples for the optional component e) are monohydroxy-functional polyalkylene oxide polyether alcohols having on statistical average 5 to 70, preferably 7 to 55, ethylene oxide units per molecule, as are accessible in a manner known per se by alkoxylation of suitable starter molecules (described e.g. in Ullmanns Encyclopädie der technischen Chemie [Ullmann's Encyclopedia of Industrial Chemistry], 4th edition, volume 19, Verlag Chemie, Weinheim pp. 31-38). These compounds are either pure polyethylene oxide ethers or mixed polyalkylene oxide ethers, in which case, however, they then contain at least 30 mol%, preferably at least 40 mol%, based on all contained alkylene oxide units, of ethylene oxide units. Particularly preferred nonionic compounds are monofunctional, mixed polyalkylene oxide polyethers which have 40 to 100 mol% ethylene oxide units and 0 to 60 mol% propylene oxide units.

For the hydrophilization it is also possible to use mixtures of anionic or potentially anionic hydrophilizing agents and nonionic hydrophilizing agents.

Polyols suitable as component f) are the polyesterpolyols, polyacrylatepolyols, polyurethanepolyols, polycarbonatepolyols, polyesterpolyacrylatepolyols, polyurethanepolyacrylatepolyols, polyurethanepolyesterpolyols, polyurethanepolyetherpolyols, polyurethanepolycarbonatepolyols, polyetherpolycarbonatepolyols and/or polyesterpolycarbonatepolyols, in particular polyesterpolyols and/or polycarbonatepolyols, known per se in polyurethane technology, especially in the polyurethane coating technology.

Polyesterpolyols are for example the polycondensates, known per se, of di- and optionally tri-, and tetraols and di- and optionally tri- and tetracarboxylic acids or hydroxycarboxylic acids or lactones. Instead of the free polycarboxylic acids, the corresponding polycarboxylic anhydrides or corresponding polycarboxylic acid esters of lower alcohols can also be used for preparing the polyesters.

Preferably, for the composition of the polyurethane or polyurethaneurea a hydrophilizing component g) is used. Compontent g) is an anionically hydrophilizing component and preferably a sulfonate.

Suitable anionically or potentially anionically hydrophilizing compounds of component g) are compounds which have at least one isocyanate-reactive group such as a hydroxyl group or amino group and at least one functionality such as e.g. $-COO^-M^+$, $-SO_3^-M^+$, $-PO(O^-M^+)_2$ where $M^+$ is for example metal cation, $H^+$, $NH_4^+$, $NHR_3^+$, where R can in each case be a C1-C12-alkyl radical, C5-C6-cycloalkyl radical and/or a C2-C4-hydroxyalkyl radical, which enters into a pH-dependent dissociation equilibrium upon interaction with aqueous media and, in so doing, can be negatively or neutrally charged. Suitable anionically or potentially anionically hydrophilizing compounds are mono- and dihydroxycarboxylic acids, mono- and dihydroxysulfonic acids, and also mono- and dihydroxyphosphonic acids and their salts. Examples of such anionic or potentially anionic hydrophilizing agents are dimethylolpropionic acid, dimethylolbutyric acid, hydroxypivalic acid, malic acid, citric acid, glycolic acid, lactic acid and the propoxylated adduct of 2-butenediol and $NaHSO_3$, as is described in DE-A 2 446 440, pages 5-9, formula I-III. Preferred anionic or potentially anionic hydrophilizing agents are those of the aforementioned type which have carboxylate or carboxylic acid groups and/or sulfonate groups.

Suitable nonionically hydrophilizing compounds of component g) are e.g. polyoxyalkylene ethers which contain at least one hydroxy or amino group, preferably at least one hydroxy group.

Examples are the monohydroxy-functional polyalkylene oxide polyether alcohols having on statistical average 5 to 70, preferably 7 to 55, ethylene oxide units per molecule, as are accessible in a manner known per se by alkoxylation of suitable starter molecules (described e.g. in Ullmanns Encyclopädie der technischen Chemie [Ullmann's Encyclopedia of Industrial Chemistry], 4th edition, volume 19, Verlag Chemie, Weinheim pp. 31-38). These compounds are either pure polyethylene oxide ethers or mixed polyalkylene oxide ethers, in which case, however, they then contain at least 30 mol%, preferably at least 40 mol%, based on all contained alkylene oxide units, of ethylene oxide units. Particularly preferred nonionic compounds are monofunctional, mixed polyalkylene oxide polyethers which have 40 to 100 mol% ethylene oxide units and 0 to 60 mol% propylene oxide units.

For the hydrophilization it is also possible to use mixtures of anionic or potentially anionic hydrophilizing agents and nonionic hydrophilizing agents.

Apart from components a), b), optionally c) to g), no further building blocks are preferably used for producing the polyurethane urea according to the invention.

The preparation of the polyurethane urea according to the invention can take place by processes known to the person skilled in the art in one or more stage(s) in homogeneous or for multistage reaction and be carried out in part in disperse phase. A dispersing, emulsifying or dissolution step preferably takes place following completely or partially carried out polyaddition from a) to g). Subsequently, a further polyaddition or modification optionally takes place in

disperse or dissolved (homogeneous) phase. In this connection, it is possible to use all processes known from the prior art, such as e.g. prepolymer mixing processes, acetone processes or melt dispersion processes. Preference is given to working in accordance with the acetone process.

Besides the polyurethane described above, the composition according to the invention can comprise further suitable film formers, which can in particular also contribute to the setting and the styling of the hair. According to the invention hair also includes eyebrows and eyelashes.

In a preferred embodiment of the film forming polyurethane the polyol component b) comprises a polyester polyol, preferably to an extend of at least 76 wt.-%, preferably to an extend of at least 80 wt.-%, or preferably to an extend of at least 85 wt.-%, based on the total weight of the polyol component b). Preferably, the film forming polyurethane is built from a polyester polyol to an extend of at least 76 wt.-%, preferably to an extend of at least 80 wt.-%, or preferably to an extend of at least 85 wt.-%, based on the total weight of all polyol components used in the building process of the film forming polyurethane. Preferably, the polyol component b) comprises in particular aliphatic polyester polyols based on aliphatic carboxylic acids and aliphatic polyols, in particular based on adipic acid and aliphatic alcohols, such as hexanediol and/or neopentyl glycol.

In a preferred embodiment of the film forming polyurethane the polyol component b) comprises $\leq$ 30 wt.-%, preferably $\leq$ 25 wt.-%, or preferably $\leq$ 20 wt.-% of a polyetherpolyol, based on the total weight of the polyol component b). Examples of preferred polyetherpolyols have been listed above. Preferably, the polyol component b) does not comprise an polyetherpolyols.

In a preferred embodiment of the film forming polyurethane the polyol component b) is a polyesterpolyol based at least partly on adipic acid, preferably in a range of from 40 to 60 wt.-%, or preferably in a range of from 45 to 58 wt.-%, or preferably in a range of from 48 to 55 wt.-%, based on the total weight of component b). Preferably, the film forming polyurethane comprises $\leq$ 30 wt.-%, or preferably $\leq$ 20 wt.-%, or preferably $\leq$ 10 wt.-% of a polyesterpolyol based on succinic acid.

In a preferred embodiment of the film forming polyurethane the polyol component b) is a polyesterpolyol based at least partly on 1,6-hexandiol, preferably in a range of from 20 to 40 wt.-%, or preferably in a range from 25 to 35 wt.-%, or preferably in a range of from 27 to 32 wt.-%.

In a preferred embodiment of the film forming polyurethane the polyol component b) is a polyesterpolyol based at least partly on neopentyl glycol, preferably in a range of from 10 to 30 wt.-%, or preferably in a range from 15 to 25 wt.-%.

In a preferred embodiment of the film forming polyurethane the polyol component b) is composed of adipic acid, 1,6-hexandiol and neopentyl glycol, preferably in a ration of adipic acid to the sum of 1,6-hexandiol and neopentyl glycol in a range of from 2:1 to 1:2, preferably in a range of from 1.5:1 to 1:1.5, or preferably in a range of from 1.1:1 to 1:1.1. Preferably, the polyol component b) is composed of adipic acid, 1,6-hexandiol and neopentyl glycol, whereby the ration of 1,6-hexandiol to neopentyl glycol is in a range of from 2:1 to 1:1.5, or preferably in a range of from 1.7:1 to 1:1.2, or preferably in a range of from 1.5:1 to 1:1.

In a preferred embodiment of the film forming polyurethane the polyol component b) has a molecular weight of $\leq$ 600 g/mol, or preferably $\leq$ 400 g/mol.

In a preferred embodiment of the film forming polyurethane the polyol component b) has a molecular weight of $\geq$ 330 g/mol, or preferably of $\geq$ 350 g/mol. Preferably, the polyol component b) has a molecular weight in a range of from 330 to 600 g/mol or preferably in a range of from 350 to 500 g/mol.

In a preferred embodiment of the film forming polyurethane the diisocyanate component a) comprises an aliphatic diisocyanates and preferably consist of at least one aliphatic diisocyanate. Examples of aliphatic diisocyanates have been given above.

In a preferred embodiment of the film forming polyurethane the diisocyanate component a) is selected from the group consisting of HDI, IPDI, H12-MDI or a mixture of at least two thereof.

A further aspect of the invention is a cosmetic formulation comprising a film forming polyurethane in a range of from 0.1 to 40 wt.-%, preferably in a range of from 0.2 to 20 wt.-%, or preferably in a range of from 0.3 to 10 wt.-%, based on the total amount of the cosmetic formulation, wherein the film forming polyurethane provides a biodegradation rate according to OECD Guideline 301 B in water of at least 50 %, preferably of at least 52 %, or preferably of at least 55 %, or preferably of at least 57 % in 28 days. In the context of the invention a cosmetic formulation and a cosmetic composition are equal in their meaning and therefore are used in both forms hereinafter.

Besides the polyurethane according to the invention described above, the cosmetic formulation according to the invention can comprise further suitable film formers.

The fraction of one or more further film formers can be from 0 to 20 wt.-% and in particular 0 to 10 wt.-%, based on the total weight of the cosmetic formulation. The further film formers can be any known to the person skilled in the art.

The composition is preferably provided, for example, in the form selected from the group consisting of an aqueous solution, an aqueous-alcoholic solution, an oily solution, oil-in-water, water-in-oil, silicone-in-water, water-in-silicone, oil-in-water-in-oil, water-in-oil-in-water emulsion, an aqueous gel, an oily gel, a pasty anhydrous product, a solid anhydrous product, a dispersion of a fatty phase in an aqueous phase in the presence of spherules. Preferably, the

composition is provided in form of an aqueous solution selected from the group consisting of an aqueous-alcoholic solution, oil-in-water, water-in-oil and water-in-silicone.

The composition can also be foamed using a propellant gas. The emulsions described above can be stabilized by an O/W, W/O or W/Si emulsifier, thickener (such as, for example, hydrodispersion) or solids (such as, for example, Pickering emulsion).

The composition can comprise one or more emulsifiers or surface-active agents.

Thus, in particular oil-in-water emulsions (O/W) according to the invention comprise preferably at least one emulsifier with an HLB (hydrophilic-lipophilic balance) value of > 7 and, if appropriate, a coemulsifier.

O/W emulsifiers can advantageously be selected from the group of nonionic, anionic, cationic or amphoteric emulsifiers to the composition.

The nonionic emulsifiers include:

a) partial fatty acid esters and fatty acid esters of polyhydric alcohols and ethoxylated derivatives thereof
b) ethoxylated fatty alcohols and fatty acids
c) ethoxylated fatty amines, fatty acid amides, fatty acid alkanolamides
d) alkylphenol polyglycol ethers (e.g. Triton® X)
e) ethoxylated fatty alcohol ethers.

Particularly advantageous nonionic O/W emulsifiers are ethoxylated fatty alcohols or fatty acids, preferably PEG-100 stearate, PEG-40 stearate, PEG-50 stearate, ceteareth-20, ceteth-20, steareth-20, ceteareth-12, ceteth-12, steareth-12, esters of mono-, oligo- or polysaccharides with fatty acids, preferably cetearyl glucoside, methylglucose distearate, glyceryl monostearates (self-emulsifying), sorbitan esters, such as, for example, sorbitan stearates (Tween® 20 and Tween® 60 from Croda), sorbitan palmitates (Span® 40, Croda), glyceryl stearyl citrates, sucrose esters, such as, for example, sucrose stearates, PEG-20 methyl glucose sesquistearate), dicarboxylic acid esters of fatty alcohol (dimyristyl tartrate).

Advantageous anionic emulsifiers are soaps (e.g. sodium or triethanolamine salts of stearic acid, glutamic acid or palmitic acid), esters of citric acid, such as glyceryl stearate citrate, fatty alcohol sulphates, and also mono-, di- and trialkyl phosphoric acid esters and ethoxylates thereof.

The cationic emulsifiers include quaternary ammonium compounds with a long-chain aliphatic radical, e.g. distearyl dimonium chloride.

The amphoteric emulsifiers include:

a. alkylaminoalkane carboxylic acids
b. betaines, sulphobetaines
c. imidazoline derivatives.

Furthermore, there are naturally occurring emulsifiers, which include beeswax, wool wax, lecithin and sterols.

Suitable coemulsifiers for the O/W emulsions which can be used for the preparation of the composition are fatty alcohols having 8 to 30 carbon atoms, monoglycerol esters of saturated or unsaturated, branched or unbranched alkanecarboxylic acids with a chain length of from 8 to 24 carbon atoms, in particular 12 to 18 carbon atoms, propylene glycol esters of saturated or unsaturated, branched or unbranched alkanecarboxylic acids with a chain length of from 8 to 24 carbon atoms, in particular 12 to 18 carbon atoms, and also sorbitan esters of saturated or unsaturated, branched or unbranched alkanecarboxylic acids with a chain length of from 8 to 24 carbon atoms, in particular 12 to 18 carbon atoms.

Particularly advantageous coemulsifiers are glyceryl monostearate, glyceryl monooleate, diglyceryl monostearate, sorbitan monoisostearate, sucrose distearate, cetyl alcohol, stearyl alcohol, behenyl alcohol, isobehenyl alcohol and polyethylene glycol(2) stearyl ether (steareth-2).

Within the context of the present invention, it may be advantageous to use further emulsifiers for the preparation of the composition according to the invention. Thus, for example, the water resistance of the preparations according to the invention can be increased. Suitable emulsifiers are, for example, alkylmethicone copolyols and alkyldimethicone copolyols, in particular cetyldimethicone copolyol, laurylmethicone copolyol, W/O emulsifiers, such as sorbitan stearate, glyceryl stearate, glycerol stearate, sorbitan oleate, lecithin, glyceryl isostearate, polyglyceryl-3 oleate, polyglyceryl-3 diisostearate, PEG-7-hydrogenated castor oil, polyglyceryl-4 isostearate, acrylate/$C_{10-30}$-alkyl acrylate crosspolymer, sorbitan isostearate, poloxamer 101, polyglyceryl-2 dipolyhydroxystearate, polyglyceryl-3 diisostearate, polyglyceryl-4 dipolyhydroxystearate, PEG-30 dipolyhydroxystearate, diisostearoyl polyglyceryl-3 diisostearate, glycol distearate and polyglyceryl-3 dipolyhydroxystearate.

In case of an O/W composition, the compositions according to the invention can furthermore contain thickeners. Advantageous thickeners are:

- Crosslinked or non-crosslinked acrylic acid or methacrylic acid homo- or copolymers. These include crosslinked homopolymers of methacrylic acid or acrylic acid, copolymers of acrylic acid and/or methacrylic acid and monomers which are derived from other acrylic or vinyl monomers, such as CI0-30 alkyl acrylates, C10-30-alkyl methacrylates and vinyl acetate.

- Thickening polymers of natural origin, for example based on cellulose, guar gum, xanthan, scleroglucan, gellan gum, rhamsan and karaya gum, alginates, maltodextrin, starch and its derivatives, carob seed flour, hyaluronic acid.

- Nonionic, anionic, cationic or amphoteric associative polymers e.g. based on polyethylene glycols and their derivatives, or polyurethanes.

- Crosslinked or non-crosslinked homopolymers or copolymers based on acrylamide or methacrylamide, such as homopolymers of 2-acrylamido-2-methylpropanesulfonic acid, copolymers of acrylamide or methacrylamide and methacryloyloxyethyltrimethylammonium chloride or copolymers of acrylamide and 2-acrylamido-2-methylpropanesulfonic acid are given.

Particularly advantageous thickeners are thickening polymers of natural origin, crosslinked acrylic acid or methacrylic acid homopolymers or copolymers and crosslinked copolymers of 2-acrylamido-2-methylpropanesulphonic acid.

Very particularly advantageous thickeners are xanthan gum, such as the products supplied under the names Keltrol® and Kelza® by CP Kelco, and guar gum, such as the products available under the name Jaguar® HP from Solvay Novecare.

Further very particularly advantageous thickeners are crosslinked homopolymers of methacrylic acid or acrylic acid which are commercially available from Noveon under the names Carbopol® 940, Carbopol® 941, Carbopol® 980, Carbopol® 981, Carbopol® ETD 2001, Carbopol® EDT 2050, Carbopol® 2984, Carbopol® 5984, Carbopol® Clear, Carbopol® Ultrez 30 and Carbopol® Ultrez 10, from 3V under the names Synthalen® K, Synthalen® L and Synthalen® MS.

Further very particularly advantageous thickeners are crosslinked polymers of acrylic acid or methacrylic acid and a C10-30-alkyl acrylate or C10-30-alkyl methacrylate and copolymers of acrylic acid or methacrylic acid and vinylpyrrolidone. Such copolymers are commercially available, for example, from Noveon under the names Carbopol® 1342, Carbopol® 1382, Carbopol® EDT 2020, Carbopol® Ultrez 20, Carbopol® Ultrez 21, Pemulen™ TR1, Pemulen™ EZ-4U or Pemulen™ TR2 and from Ashland under the names Ultrathix P-100 (INCI: Acrylic Acid/VP Crosspolymer).

Very particular advantageous thickeners are crosslinked copolymers of 2-acrylamido-2-methylpropanesulphonic acid. Such copolymers are available, for example, from Clariant under the names Aristoflex® AVC or Aristoflex® AVS (INCI: Ammonium Acryloyldimethyltaurate/VP Copolymer), Aristoflex® Silk (INCI : Sodium Polyacryloyldimethyltaurate), Aristoflex® BLV or Aristoflex ® HMB (INCI: Ammonium Acryloyldimethyltaurate/Beheneth-25 Methacrylate Crosspolymer).

These thickeners are preferably present in the composition in a concentration in the range of from about 0 to 2 wt.-%, preferably 0 to 1 wt.-%, based on the total weight of the composition.

Preferably, the composition is a water-in-oil or water-in-silicone emulsion. Preference is given to water-in-oil (W/O) or water-in-silicone emulsions (W/Si) which comprise one or more silicone emulsifiers (W/S) with an HLB value of $\leq 8$ or one or more W/O emulsifiers with an HLB value of $\leq 7$ and optionally one or more O/W emulsifiers with an HLB value of $\geq 10$.

The silicone emulsifiers can advantageously be selected from the group selected from alkyldimethicone copolyols, such as, for example, cetyl PEG/PPG 10/1 dimethicone copolyol (ABIL® EM 90 from Evonik) or lauryl PEG/PPG-18/18 dimethicones (Dow Corning® 5200 from Dow Corning Ltd.) and dimethicone copolyols, such as, for example, PEG-10 dimethicones (KF-6017 from Shin Etsu), PEG/PPG- 18/18 dimethicones (Dow Corning 5225C from Dow Corning Ltd.), PEG/PPG-19/19 dimethicones (Dow Corning BY-11 030 from Dow Corning Ltd.), trimethylsilylamodimethicones or mixtures of at least two thereof.

The W/O emulsifiers with an HLB value of < 7 can advantageously be selected from the fol-lowing group: fatty alcohols having 8 to 30 carbon atoms, monoglycerol esters of saturated and/or unsaturated, branched and/or unbranched alkanecarboxylic acids of chain length of from 8 to 24, in particular 12 18 carbon atoms, diglycerol esters of saturated and/or unsaturated, branched and/or unbranched alkanecarboxylic acids of chain length from 8 to 24, in particular 12 18, carbon atoms, monoglycerol ethers of saturated and/or unsaturated, branched and/or unbranched alcohols of chain length of from 8 to 24, in particular 12 18, carbon atoms, diglycerol ethers of saturated and/or unsaturated, branched and/or unbranched alcohols of chain length from 8 to 24, in particular 12 18, carbon atoms, propylene glycol esters of saturated and/or unsaturated, branched and/or unbranched alkanecarboxylic acids of chain length from 8 to 24, in particular 12 18, carbon atoms, and also sorbitan esters of saturated and/or unsaturated, branched and/or unbranched alkanecarboxylic acids of chain length from 8 to 24, in particular 12 18, carbon atoms or a mixture of at least two thereof.

Particularly advantageous W/O emulsifiers are: glyceryl monostearate, glyceryl monoisostearate, glyceryl monomyristate, glyceryl monooleate, diglyceryl monostearate, diglyceryl monoisos-tearate, propylene glycol monostearate, propylene glycol monoisostearate, propylene glycol monocaprylate, propylene glycol monolaurate, sorbitan monoisostearate, sorbitan monolaurate, sorbitan monocaprylate, sorbitan monoisooleate, sucrose distearate, cetyl alcohol, stearyl alcohol, arachidyl alcohol, behenyl alcohol, isobehenyl alcohol, selachyl alcohol, chimyl alcohol, polyethylene glycol(2) stearyl ether (steareth-2), glyceryl monolaurate, glyceryl monocaprate and glyceryl monocaprylate or mixtures of at least two thereof.

Further possible W/O emulsifiers are selected from the group consisting of polyglyceryl-2 dipolyhydroxystearate, PEG 30 dipolyhydroxystearate, cetyldimethicone copolyol and polyglyceryl-3 diisostearate or mixtures of at least two thereof.

The O/W emulsifiers with an HLB value of > 10 can advantageously be selected from the group consisting of lecithin, trilaureth-4 phosphate, polysorbate-20, polysorbate-60, PEG 22 dodecyl glycol copolymer, sucrose stearate and sucrose laurate or mixtures of at least two thereof.

An oil thickener can advantageously be used for stabilizing the W/O emulsion according to the invention against sedimentation or flocculation of the water droplets.

Particularly advantageous oil thickeners are organomodified clays, such as organomodified bentonites (Bentone® 34 from Elementis Specialtis), organomodified hectorites (Bentone® 27 and Bentone® 38 from Elementis Specialtis) or organomodified montmorillonite, hydrophobic pyrogenic silica, where the silanol groups are substituted by trimethylsiloxy groups (AEROSIL® R812 from Evonik) or with dimethylsiloxy groups or polydimethylsiloxane (AEROSIL® R972, AEROSIL® R974 from Evonik), magnesium or aluminium stearate, or styrene copolymers, such as, for example, styrene-butadiene-styrene, styrene-isopropene-styrene, styrene-ethylene/butene-styrene or styrene-ethylene/propene-styrene.

The thickener for the fatty phase can be present in an amount of from 0.1 to 5 wt.-%, based on the total weight of the emulsion, and better 0.4 to 3 wt.-%.

The aqueous phase can also comprise stabilizers. The stabilizer can be, for example, sodium chloride, magnesium chloride or magnesium sulphate and mixtures thereof.

Oils can be used in W/O, W/Si and O/W emulsions.

If present, the fatty phase of the composition according to the invention can comprise one non-volatile oil and/or volatile oils and waxes. The O/W composition comprises advantageously 0.01 to 45 wt.-% of oils, based on the total weight of the composition, and particularly advantageously 0.01 to 20 wt.-% of oils. The W/O or W/Si composition advantageously comprises at least 20 wt.-% of oils, based on the total weight of the composition.

The non-volatile oil is advantageously selected from the group of mineral, animal, vegetable or synthetic origin, polar or nonpolar oils and mixtures thereof.

The polar oils are advantageously selected from the group:

a. esters of saturated and/or unsaturated, branched and/or unbranched alkanecarboxylic acids of chain length from 3 to 30 carbon atoms and saturated and/or unsaturated, branched and/or unbranched alcohols of chain length from 3 to 30 carbon atoms, or mixtures of at least two thereof;

b. esters of aromatic carboxylic acids and saturated and/or unsaturated, branched and/or un-branched alcohols of chain length from 3 to 30 carbon atoms, or mixtures of at least two thereof.

Such ester oils can then advantageously be selected from the group:
isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, isotridecyl isononanoate, 2-ethylhexyl palmitate, 2-ethylhexyl laurate, 2-ethylhexyl isostearate, 2-hexyldecyl stearate, 2-octyldodecyl palmitate, 2-ethylhexyl cocoate, oleyl oleate, oleyl erucate, erucyl oleate, erucyl erucate, dicaprylyl carbonate (Cetiol® CC) and cocoglycerides (Myritol® 331), and also synthetic, semisynthetic and natural mixtures of such esters, e.g. jojoba oil, or mixtures of at least two thereof.

c. alkyl benzoates C12-15-alkyl benzoate (Cosmacol EBL von Sasol Performance Chemicals) or 2-phenylethyl benzoate (X-Tend® 226 from Ashland)

d. lecithins and the fatty acid triglycerides, namely the triglycerol esters of saturated and/or unsaturated, branched and/or unbranched alkanecarboxylic acids of chain length from 8 to 24, in particular 12 to 18 carbon atoms. For example, the fatty acid triglycerides can be selected from the group of cocoglyceride, olive oil, sunflower oil, soybean oil, peanut oil, rapeseed oil, almond oil, palm oil, coconut oil, castor oil, wheat germ oil, grapeseed oil, safflower oil, evening primrose oil, macadamia nut oil, apricot kernel oil, avocado oil and the like, or mixtures of at least two thereof.

e. of dialkyl ethers and dialkyl carbonates, e.g. dicaprylyl ether (Cetiol® OE from BASF) and/or dicaprylyl carbonate (for example Cetiol® CC from BASF) are advantageous

f. of saturated or unsaturated, branched or unbranched alcohols, such as, for example, octyldodecanol.

The non-volatile oil can likewise advantageously also be a nonpolar oil which is selected from the group of branched and unbranched hydrocarbons, in particular mineral oil, vaseline oil, paraffin oil, squalane and squalene, polyolefins, for example polydecenes, hydrogenated polyisobutenes, C13-16 isoparaffin and isohexadecane or mixtures of at least two thereof.

The nonpolar non-volatile oil can be selected among the non-volatile silicone oils.

Of the non-volatile silicone oils, the polydimethylsiloxanes (PDMS), which are optionally phenylated, such as phenyltrimethicone, or are optionally substituted with aliphatic and/or aromatic groups or with functional groups, for example hydroxyl groups, thiol groups and/or amino groups; polysiloxanes modified with fatty acids, fatty alcohols or polyoxyalkylenes and mixtures thereof can be given.

Particularly advantageous oils are 2-ethylhexyl isostearate, octyldodecanol, isotridecyl isononanoate, isoeicosane, 2-ethylhexyl cocoate, C12 15 alkyl benzoate, caprylic/capric triglyceride, dicaprylyl ether, mineral oil, dicaprylyl carbonate, cocoglycerides, butylene glycol dicaprylate/dicaprate, hydrogenated polyisobutenes, cetaryl isononanoates, isodecyl neopentanoates, squalane, C13 16 isoparaffin or mixtures of at least two thereof.

The composition according to the invention preferably comprises a wax.

Within the context of the present specification, a wax is defined as a lipophilic fatty substance which is solid at room temperature (25°C) and exhibits a reversible solid/liquid change in state at a melting temperature between 30°C and 200°C. Above the melting point, the wax becomes low viscosity and is miscible with oils.

The wax is advantageously selected from the group consisting of natural waxes, such as, for example, cotton wax, carnauba wax, candelilla wax, esparto wax, Japan wax, Montan wax, sugarcane wax, beeswax, wool wax, shellac, microwaxes, ceresine, ozokerite, ouricury wax, cork fibre wax, lignite waxes, berry wax, shea butter or synthetic waxes, such as paraffin waxes, polyethylene waxes, waxes produced by Fischer-Tropsch synthesis, hydrogenated oils, fatty acid esters and glycerides which are solid at 25°C, silicone waxes and derivatives (alkyl derivatives, alkoxy derivatives, and/or esters of polymethylsiloxane) or mixtures of at least two thereof. The waxes can be present in the form of stable dispersions of colloidal wax particles which can be prepared by known processes, for example as in "Microemulsions Theory and Practice", L.M. Prince Ed., Academic Press (1977), pages 21-32.

Waxes may be present in amounts of from 0 to 10 wt.-%, based on the total weight of the composition, and preferably 0 to 5 wt.-%.

The composition preferably comprises a volatile oil which is selected from the group of volatile hydrocarbon oils, siliconized oils or fluorinated oils.

The volatile oil can be present in an amount of from 0 to 25 wt.-%, based on the total weight of the emulsion, preferably 0 to 20 wt.-% and even more preferably 0 to 15 wt.-%.

Within the context of the present specification, a volatile oil is oil which, upon contact with the skin at room temperature and atmospheric pressure, evaporates in less than one hour. The volatile oil is liquid at room temperature and, at room temperature and atmospheric pressure, has a vapour pressure of from 0.13 to 40 000 Pa (10-3 to 300 mm Hg), preferably 1.3 to 13 000 Pa (0.01 to 100 mmHg) and particularly preferably 1.3 to 1300 Pa (0.01 to 10 mmHg) and a boiling point of from 150 to 260°C and preferably 170 to 250°C.

Hydrocarbon oil is understood as an oil which is formed essentially from carbon atoms and hydrogen atoms and optionally oxygen atoms or nitrogen atoms and contains no silicon atoms or fluorine atoms, where it may also consist of carbon atoms and hydrogen atoms; however, it can also contain ester groups, ether groups, amino groups or amide groups.

Siliconized oil is understood as oil which contains at least one silicon atom and in particular Si-O groups.

Fluorinated oil is to be understood as oil which contains at least one fluorine atom.

The volatile hydrocarbon oil according to the invention can be selected from the hydrocarbon oils with a flash point of from 40 to 102°C, preferably 40 to 55°C and even more preferably 40 to 50°C.

For example, the volatile hydrocarbon oils are those with 8 to 16 carbon atoms and mixtures thereof, in particular branched C8-16-alkanes, such as the isoalkanes (which are also referred to as isoparaffins) with 8 to 16 carbon atoms, isododecane, isodecane, isohexadecane and, for example, the oils which are supplied under the tradenames Isopars® or Permetyls®; and the branched C8-16-esters, such as isohexyl neopentanoate or mixtures of at least two thereof.

The volatile hydrocarbon oils such as isododecane, isodecane and isohexadecane are particularly advantageous.

The volatile siliconized oil is preferably selected from the siliconized oils with a flash point of from 40 to 102°C, preferably a flash point above 55°C and at most 95°C and particularly preferably in the range from 65 to 95°C.

For example, the volatile siliconized oils are straight-chain or cyclic silicone oils having 2 to 7 silicon atoms,

where these silicones optionally contain alkyl or alkoxy groups having 1 to 10 carbon atoms.

The volatile siliconized oils are preferably selected from the group consisting of octamethylcyclotetrasiloxane, decamethylcyclopentasiloxane, dodecamethylcyclohexasiloxane, heptamethylhex-yltrisiloxane, heptamethyloctyltrisiloxane, hexamethyldisiloxane, octamethyltrisiloxane, decame-thyltetrasiloxane, dodecamethylpentasiloxane or mixtures of at least two thereof.

The volatile fluorinated oil generally has no flash point.

For example, the volatile fluorinated oils are nonafluoroethoxybutane, nonafluoromethoxybutane, decafluoropentane, tetradecafluorohexane, dodecafluoropentane or mixtures of at least two thereof.

Preferably, the composition comprises water and optionally a water-miscible organic solvent.

The water used in the composition preferably is selected from the group consisting of a blossom water, pure demineralized water, mineral water, thermal water and seawater or mixtures of at least two thereof.

In the case of an O/W composition, the water fraction is preferably in the range from 40 to 95 wt.-%, preferably in the range from 50 to 90 wt.-%, very particularly in the range from 60 to 80 wt.-%, based on the total weight of the composition. In the case of a W/O composition, the water fraction is preferably in the range from 0 to 60 wt.-%, preferably in the range from 10 to 50 wt.-%, very preferably in the range from 30 to 50 wt.-%, based on the total weight of the composition.

In the case of an aqueous composition, the water fraction is preferably in the range from 40 to 95 wt.-%, preferably in the range from 50 to 90 wt.-%, very particularly in the range from 60 to 80 wt.-%, based on the total weight of the composition. In the case of an aqueous-alcoholic solution, the water fraction is preferably in the range from 20 to 80 wt.-%, preferably in the range from 25 to 70 wt.-%, very preferably in the range from 30 to 60 wt.-%, based on the total weight of the composition.

Preferred solvents are, for example, the aliphatic alcohols with CI-4 carbon atoms, such as etha-nol and isopropanol; polyol and derivatives thereof, such as propylene glycol, dipropylene glycol, butylene-1,3 glycol, polypropylene glycol, glycol ethers such as alkyl (CI-4) ethers of mono-, di- or tripropylene glycol or mono-, di- or triethylene glycol, and mixtures thereof.

The quantitative fraction of the solvent or solvents in the composition are preferably in the range of from 0 to 25 wt.-%, or preferably 0 to 15 wt.-%, based on the total weight of the composition. In case of an aqueous-alcoholic solution the solvent content is preferably in the range of 10 to 98 wt.-%, preferably in the range from 15 to 90 wt.-%, or preferably in the range from 20 to 80 wt.-%, based on the total weight of the composition

Optionally, conventional additives can likewise be present in the composition, for example in order to impart certain modifying properties to it. These may be for example silicones or silicone derivatives, wetting agents, humectants, softeners such as glycerol, glycol and phthalic esters and ethers, fragrances and perfumes, UV absorbers, actives, dyes, pigments, and other colorants, anticorrosive agents, neutralizing agents, antioxidants, anti-sticking agents, combining agents and conditioning agents, antistatic agents, shine agents, preservatives, proteins and derivatives thereof, amino acids, vitamins, emulsifiers, surface-active agents, viscosity modifiers, thickeners and rheology modifiers, gelling agents, opacifiers, stabilizers, surfactants, sequestrants, complexing agents, pearlizing agents, esthetic boosters, fatty acids, fatty alcohols, triglycerides, botanical extracts, clarifying auxiliaries and film formers.

If additives are added to the cosmetic formulation or composition, the additives are preferably present in a concentration of about 0.001% to 60 wt.-%, preferably 0.01% to 40 wt.-%, based on the total weight of the composition.

In the context of the invention, active ingredients are defined as elements or chemical compounds which have a specific effect on living systems, more particularly prions, viruses, bacteria, cells, fungi and organisms. Examples of these active ingredients are described in the patent application WO 2016/116403 A1, especially those described on page 16 line 23 to page 31 line 9. Examples of suitable UV absorbers are described in the patent application WO 2009/11813 A1, especially on pages 27 line 17 to page 31 line 16. Especially for hair care applications film formers as describes in WO 2009/118105 A1 on page 23 line 30 to 33 line 19.

Within the context of the present invention, the composition is differentiated in particular according to their consistency: solid or foam (solid), cream (viscous), lotion or milk (flowable), paste or gels (semisolid), oils, and also balm, serum, ointment or aqueous solutions (liquid).

The compositions according to the invention can advantageously be present in a pump spray or aerosol packaging. They can also advantageously be foamed using a propellant gas. Correspondingly, pump sprays, aerosol packagings and foam dispensers which contain the composition according to the invention are likewise part of the invention.

Preferred propellant gases are hydrocarbons such as propane, isobutane and n-butane, and mixtures thereof. Compressed air, carbon dioxide, nitrogen, nitrogen dioxide and dimethyl ether, as well as mixtures of all of these gases can likewise be used.

The person skilled in the art is naturally aware that there are propellant gases that are nontoxic per se which would in principle be suitable for realizing the present invention in the form of aerosol preparations but which nevertheless should be dispensed with on account of an unacceptable impact on the environment or other accompanying phenomena. These are in particular fluorocarbons and chlorofluorocarbons (CFCs) such as e.g. 1,2-difluoroethane (propellant 152 A).

A further aspect of the invention is a use of the film forming polyurethane according to the invention in a cosmetic formulation to increase the biodegradability of the cosmetic formulation.

The polyurethane according to the invention is preferably used in cosmetics formulations, preferably in the field of nail varnish, mascara, skin care, hair cosmetics, particularly preferably in the field of hairstyling cosmetics. The cosmetic formulation preferably comprises the film forming polyurethane according to the invention in an amount of from 0.1 to 40 wt.-%, or preferably of from 0.2 to 35 wt.-%, or preferably of from 0.5 to 20 wt.-%, based on the total weight of the cosmetic formulation. Preferably, as further ingredients of the cosmetic formulation mainly biodegradable ingredients are used. Preferably, the cosmetic formulation has a total biodegradation rate of ≥ 40 %, preferably of ≥ 50 %, preferably ≥ 60%, according to OECD Guideline 301 B in water in 28 days.

A further aspect of the invention is a method for shaping hairstyles or treating hair, styling nails or treating skin in which a film forming polyurethane according to the invention or a cosmetic formulation according to the invention is applied to hair, nails or skin. Preferably, the method comprises at least the following steps:

I) optionally wetting the hair;

II) optionally cleaning the hair, nails or skin;

III) optionally forming the hair into a hairstyle;

IV) applying the film forming polyurethane or the cosmetic formulation to hair, nails or skin;

V) optionally forming the hair into a hairstyle or drying the nail or skin surface.

In the following only the details of steps I) to V) for hairstyling is described as an example. The styling of nails or the skin can be done according to known methods to apply nail varnish to nails or skin car compositions to skin.

In the optional step I) the hair is wetted preferably by rinsing water over the hair or by dipping the hair into water. In the optional step II) the hair is preferably cleaned by a conventional shampoo for at least 1 minute, preferably for at least 2 minutes. In the optional step III) the hair might be formed by different means and into different forms. For example the hair might be curled by hair rollers, by a brush or any other curls forming means. Alternatively, curly hair might be smoothened by an iron. In step IV) the film forming polyurethane or the cosmetic formulation comprising the film forming polyurethane is applied to the hair. The application of the film forming polyurethane or the cosmetic formulation comprising can be performed by any means the person skilled in the art would select for application thereof to hair. Preferably, the film forming polyurethane or the cosmetic formulation is applied to the hair homogeneously. Preferably, after application the film forming polyurethane or the cosmetic formulation comprising is left on the hair for a period of from 1 second to 60 minutes, or preferably of from 10 second to 50 minutes, or preferably of from 1 to 20 minutes, or preferably of from 2 to 10 minutes. In optional step V) the hair might be formed preferably as described for step III). Preferably, only one forming step, either step III) or step V) are performed.

If the film forming polyurethane is part of a cosmetic formulation which is suitable for skin care, nail varnish or mascara the skin, nails or eyebrows can be treated in step IV) by conventional methods of applying the cosmetic formulation to those surfaces.

## Experimental Part

### Test methods

**Determination of the biodegradation of the polymers according to '$CO_2$-Evolution-Test' (OECD Guideline 301 B in the version of 17.07.92)**.

The test item and polyvalent inoculum (sewage microorganisms from a sewage treatment plant working with predominantly domestic sewage, see below) were incubated at 20.3 °C to 21.4 °C. $CO_2$ released from the test item was trapped as $BaCO_3$ using a trap system described in the indicated guideline (OECD Guideline 301 B) and the study plan in § 8.4.4 of the guideline. The degradation was followed by $CO_2$ analysis. After reference to blank control, the total amount of $CO_2$ produced by the test item was determined for the test period and calculated as a percentage of the total $CO_2$ that the test item, based on its carbon content, could have theoretically produced (%$TCO_2$).

The results presented in Table 1 are the degradation rates of the best out of the two measured test items. The results were only considered as valid when the degradation of the two test items at any time of the test did not differ more than 20%.

Study plan

Tests shown in Figures 1 to 3 were performed using a setup and sludge described in the OECD 301 B guideline for the assessment of aerobic readily biodegradability in domestic waste water. This method is both applicable to substance soluble and poorly soluble in water. The following film forming polymers typically used for hair styling were tested: two inventive polyester based polyurethanes (according to INCI: Polyurethane-48 and Polyurethane-34), a polyacrylate copolymer (INCI: Octylacrylamide/Acrylates/Butylaminoethyl methacrylate Copolymer), and a polyvinylpyrrolidone (INCI: PVP) polymer. The acrylate copolymer was fully neutralized using sodium hydroxide prior testing in order to be fully solubilized without adding any carbon or nitrogen not bound to the backbone that could otherwise influence the results. The other polymers were used without pretreatment. The two inventive polyurethanes used are slightly anionic and contain sulfonic acid sodium salts in the polymer chain.

To perform the test according to the study plan the test sample is placed in a bottle containing a polyvalent inoculum (bacterial sludge from a sewage treatment plant treated predominantly domestic sewage) and incubated at 21°C. During the test period the solutions are stirred using a magnetic stirrer. The degradation is followed by analysis of released $CO_2$. The $CO_2$ was collected using a $Ba(OH)_2$ trap and titrated using a HC1 solution and phenolphthalein as a color indicator, following the procedure described in the guideline. The amount of $CO_2$ produced during degradation of the test item is expressed as a percentage of the theoretical $CO_2$ amount that the test items would have produced if all of its carbon atoms would be turned into $CO_2$.

Two control samples were run in parallel to the test sample to validate the measurement. To confirm adequate sludge activity, sodium benzoate, an easily biodegradable ingredient is measured. Its successful biodegradation indicates normal activity of the sludge. The sodium benzoate control is compulsory to confirm the validity of the test. The second control sample (toxicity control), is composed of the sodium benzoate together with the test item. The measurement of the toxicity control sample indicates whether any side products that are toxic towards microorganisms are released upon degradation of the test item. Release of toxic side products would be indicated by a lower level of $CO_2$ release for the toxicity control sample compared to the sodium benzoate sample.

It is also worth noting that levels of 100% biodegradation are usually not reached, since some carbon is used by microorganisms to generate new biomass. This can be seen in the 80% level of the reference of sodium benzoate which is the maximum that could be reached in a biodegradation test.

The obtained biodegradation results are shown in Figure 1 and 2. Figure 1 shows the degradation curves for Polyurethane-35, the reference sample (sodium benzoate), and the toxicity control sample (sodium benzoate + Polyurethane-35). The three experiments result in steady $CO_2$ evolution curves with no drop in $CO_2$ emission during the test period. This observation suggests that neither the polymer nor its degradation products show toxic effects on microorganisms.

EXAMPLES

Inventive Examples N° 1 to 4: polyurethanes based on at least 82.2 wt.-% (based on solid content) of polyester A

with Polyester A = copolyester of adipic acid, hexane diol and neopentyl glycol, where the ratio of these is 51.8 wt.-% : 29.8 wt.-% : 18.4 wt.-% with a molecular weight of 1700 g/mol

Polymer 1: polyurethane based on 82.2 wt.-% polyester A and Hexamethylene diisocyanate, in the form of a dispersion of 40 wt.-% polymer in water. Also contains 1.5% Dermosoft OMP (of Evonik Dr. Straetmans GmbH, Germany) additive.

Polymer 2: polyurethane based on 81.6 wt.-% polyester A and Hexamethylene diisocyanate, in the form of a dispersion of 40 wt.-% polymer in water.

Polymer 3: polyurethane based on 76.2 wt.-% polyester A and Dicyclohexyl methane diisocyanate, In the form of a dispersion of 40 wt.-% polymer in water.

Polymer 4: polyurethane based on 77.3 wt.-% polyester A and Isophorone diisocyanate, in the form of a dispersion of 30 wt.-% polymer in water.

Non-inventive Examples

Example N° 5 & 6: non-polyurethanes, radical polymers commonly used in cosmetics and having poor biodegradation ($\leq$ 20%)

Polymer 5: Amphomer: Commercial polyacrylate copolymer (INCI: Octylacrylamide / Acrylates / Butylaminoethyl methacrylate Copolymer) powder. The polymer was neutralised with NaOH until fully solubilized in water prior to the study.
Polymer 6: Polyvinyl pyrrolidone (INCI: Polyvinylpyrrolidone) powder. The polymer is water soluble and was dissolved in water prior to the biodegradation study.

Non-inventive Examples 7 to 11

Examples N° 7 and 8: polyurethanes based on polyether precursors show low biodegradation level

Polymer N° 7: Polyether based polyurethane, based on 80.5% polytetramethylene glycol and a mixture of hexamethylene diisocyanate and isophorone diisocyanate, in the form of a dispersion of 50% polymer in water
Polymer N° 8: Polyether based polyurethane, based on 62.5 wt.-% polytetramethylene glycol and a mixture of Isophorone diisocyanate and 4,4'-Methylenbis(cyclohexylamin), in the form of an 40% solution in ethanol. The ethanol was removed prior to the biodegradation test.

Examples N° 9: polyurethanes based on at least 64.3 wt.-% of polyester A

Polymer N° 9: polyurethane based on 64.3 wt.-% polyester A and and Dicyclohexyl methane diisocyanate, in the form of a dispersion of 40 wt.-% polymer in water.

Examples N° 10 and 11: polyurethanes based on at least 76.4 wt.-% of polyester B

With Polyester B = copolyester of succinic acid, butane diol and neopentyl glycol (52.3 wt.-% : 27.0 wt.-% : 20.7 wt.-%) with a molecular weight of 1675 g/mol

Polymer N° 10: Polyurethane based on 77.0 wt.-% Polyester B and Isophorone diisocyanate, in the form of a dispersion of 30 wt.-% polymer in water.
Polymer N° 11: Polyurethane based on 76.4 wt.-% Polyester B and Dicyclohexyl methane diisocyanate in the form of a dispersion of 40 wt.-% polymer in water.

Biodegration Results of polymers 1 to 11 wherein Examples 1 to 4 are inventive

| Test duration (days) | Ex. 1* | Ex. 2* | Ex. 3* | Ex. 4* | Ex. 5 | Ex. 6 |
|---|---|---|---|---|---|---|
| 0 | 0 | 0 | 0 | 0 | 0 | 0 |
| 4 | 1 | 1 | 3 | 1 | 0 | 2 |
| 8 | 3 | 9 | 16 | 13 | 0 | 6 |
| 12 | 7 | 20 | 27 | 26 | 0 | 11 |
| 15 | 13 | 32 | 39 | 38 | 0 | 16 |
| 19 | 24 | 42 | 46 | 45 | 0 | 19 |
| 22 | 35 | 48 | 50 | 48 | 0 | 23 |
| 28 | 50 | 56 | 55 | 51 | 0 | 29 |
| 29 | 59 | 60 | 56 | 53 | 0 | 33 |

| Test duration (days) | Ex. 7 | Ex. 8 | Ex. 9 | Ex. 10 | Ex. 11 |
|---|---|---|---|---|---|
| 0 | 0 | 0 | 0 | 0 | 0 |
| 4 | 1 | 1 | 0 | 1 | 1 |
| 8 | 2 | 4 | 1 | 2 | 2 |
| 12 | 5 | 6 | 5 | 3 | 3 |
| 15 | 9 | 10 | 9 | 6 | 4 |
| 19 | 11 | 12 | 10 | 7 | 6 |
| 22 | 12 | 16 | 12 | 10 | 8 |
| 28 | 15 | 18 | 13 | 13 | 10 |
| 29 | 15 | 20 | 14 | 17 | 14 |

Table 1: Biodegration Results of different polymers (* inventive examples)

As can be seen only inventive Examples 1 to 4 provide a biodegradability of at least 50 % after 28 days. All other examples, even polyurethane film formers of example 10 and 11 do not reach these high values. Thus, it was not foreseeable that the polyurethane film formers according to the invention would provide these differing biodegradability values compared to other film formers used in cosmetic formulations. By using these biodegradable polyurethane film formers cosmetic compositions with a high biodegradability could be provided.

The following figures show results of biodegradation experiments and results of different polymers in cosmetic applications:

Fig. 1 Diagram of the biodegradation of an PUD according to the invention in form of polyurethane-48 in comparison to a control (sodium benzoate) and toxicology control (Polyurethane-48 and sodium benzoate)

Fig. 2 Diagram of the biodegradation of two inventive polyurethanes, PVP and an acrylate copolymer

Fig. 3 Diagram of the high humidity (21°C, 90% rel. Hum.) curl retentions of Caucasian hair treated with a 2%-wt polymer solution according to the invention

Fig. 4 Photograph of curly hair treated by different polymers to visualize curl retention capabilities of the different polymers

In Figure 1 the results of the biodegradation as described above in connection with Table 1, where Example 2 comprises Polyurethane-34 (PU-34) and Example 4 comprises Polyurethane-48 (PU-48). It can be seen that the curves of the Benzoate control and of the Polyurethane-48 (PU-48) which corresponds to Example 4 of Table 1 have the same slope which is an indication that the microorganisms that are responsible for the biodegradation are not killed by the PU-48 or any of its degradation products that could be formed during the test duration.

In Figure 2 a similar behavior to the biodegradation of PU-48 as shown in Figure 1 can be seen for Polyurethane-34 (PU-34) which corresponds to Example 2 of Table 1. Compared to the high biodegradation values of the two inventive polymers, the polymers designated as PVP (polyvinylpyrrolidone, according to INCI: PVP) and acrylate copolymer show much less or even no biodegradability.

In Figure 3 an application of the two inventive PUs, namely PU-34 (as Example 2 in Table 1) and PU-34 (as Example 4 in Table 1) in a cosmetic application compared to the behavior of the acrylate copolymer (according to INCI: Octylacrylamide / Acrylates / Butylaminoethyl methacrylate Copolymer) is shown. The application is in the field of hair styling, especially in curl retention properties of the polymers. The diagram in Figure 3 shows that the two PU dispersions show a better curl retention on Caucasian hair at 21°C at 90% rel. humidity than curled hair treated with acrylate copolymer. The samples of Caucasian hair were treated as follows:
In order to test the performance of the investigated film formers on hair, curls were prepared by treating undamaged Caucasian hair strands with a 2 wt.-% film former solution. The solutions were applied on wet hair (1 g solution / 1 g hair). The strands were curled around a curler, blow dried for 3 minutes at 75°C, and left to dry over night at room temperature under controlled 55% relative humidity.

A first set of curls was used to measure the high humidity curl retention of the polymer films. Curls were placed in a humidity chamber for 5 hours at 21 °C and 90% relative humidity. The percentage of curl retention over time was calculated as follow:

$$\%\ \text{Curl Retention} = 100 \text{ x } \frac{lenght\ of\ uncurled\ hair - lenght\ of\ curl\ at\ time\ t}{lenght\ of\ uncurled\ hair - start\ lenght\ of\ curl}$$

Results are depicted in Figure 3. Both polyurethanes achieved curl retention levels above 65% at 300 minutes, whereas the acrylate copolymer just show a retention below 60% at 300 minutes. The polyurethanes achieved a higher retention of the curl shape, indicating less plasticization of the film by permeated water.

A second set of curls was used to test the water resistance of the polymer film. In this experiment, the curls were dipped in warm (38°C) soft water for 30 seconds, then taken out, and left to dry at room temperature and 55% relative humidity in a hanging state. The photographs of the obtained hair strands are shown Figure 4. After contact with water, hair treated with PVP fully lost its shape, while hair treated with polyacrylate and the poylurethanes maintained a curly shape, indicating better adhesion of the polymer film to the hair surface. Of all curls, the one treated with polyurethane-48 (Ex. 4 of Table 1) showed the best shape retention followed by polyurethane-34 (Ex. 2 of Table 1).

## Claims

1. A film forming polyurethane dispersion comprising a biodegradable polyurethane which is built from at least the following components:

   a) at least one aliphatic, araliphatic and/or cycloaliphatic polyisocyanate, with an NCO functionality in a range of from 1.8 to 4, preferably of from 2 to 3.
   b) at least one polyol having a number-average molecular weight Mn of ≥ 330 and ≤ 6000 g/mol, or preferably of ≥ 350 and ≤ 5000 g/mol and a hydroxyl functionality of ≥ 1.5 and ≤ 4,
   c) optionally at least one amino-functional compound which has at least two isocyanate-reactive amino groups,
   d) optionally at least one alcohol which has at least two hydroxyl groups and a molar mass of ≥ 62 and ≤ 399 g/mol,
   e) optionally at least one compound which has one group that is reactive towards isocyanate groups,

   wherein the polyurethane provides a biodegradation rate according to OECD Guideline 301 B in water of at least 50 % in 28 days.

2. The film forming polyurethane according to claim 1, wherein the polyol component b) comprises a polyester polyol, preferably to an extend of at least 76 wt.-%, preferably to an extend of at least 80 wt.-%, based on the total weight of the polyol component b).

3. The film forming polyurethane according to any of claims 1 or 2, wherein the polyol component b) comprises ≤ 30 wt.-%, preferably ≤ 25 wt.-%, or preferably ≤ 20 wt.-% of a polyetherpolyol, based on the total weight of the polyol component b).

4. The film forming polyurethane according to any of the preceding claims, wherein the polyol component b) is a polyesterpolyol based at least partly on adipic acid, preferably in a range of from 40 to 60 wt.-%, or preferably in a

range from 45 to 58 wt.-%.

5. The film forming polyurethane according to any of the preceding claims, wherein the polyol component b) is a polyesterpolyol based at least partly on 1,6-hexandiol, preferably in a range of from 20 to 40 wt.-%, or preferably in a range from 25 to 35 wt.-%.

6. The film forming polyurethane according to any of the preceding claims, wherein the polyol component b) is a polyesterpolyol based at least partly on neopentyl glycol, preferably in a range of from 10 to 30 wt.-%, or preferably in a range from 15 to 25 wt.-%.

7. The film forming polyurethane according to the preceding claims the polyol component b) is composed of adipic acid, 1,6-hexandiol and neopentyl glycol, preferably in a ration of adipic acid to the sum of 1,6-hexandiol and neopentyl glycol in a range of from 2:1 to 1:2, preferably in a range of from 1.5:1 to 1:1.5, or preferably in a range of from 1.1:1 to 1:1.1.

8. The film forming polyurethane according to any of the preceding claims, wherein the polyol component b) has a molecular weight of ≤ 600 g/mol, or preferably of ≤ 400 g/mol.

9. The film forming polyurethane according to any of the preceding claims, wherein the polyol component b) has a molecular weight of ≥ 330 g/mol, or preferably of ≥ 350 g/mol.

10. The film forming polyurethane according to any of the preceding claims, wherein the diisocyanate component a) comprises an aliphatic diisocyanates and preferably consist of at least one aliphatic diisocyanate.

11. The film forming polyurethane according to any of the preceding claims, wherein the diisocyanate component a) is selected from the group consisting of HDI, IPDI, H12-MDI or a mixture of at least two thereof.

12. A Cosmetic formulation comprising a film forming polyurethane in an range of 0.1 to 40 wt.-%, based on the total amount of the cosmetic formulation, wherein the film forming polyurethane provides a biodegradation rate according to OECD Guideline 301 B in water of at least 50 %, preferably of 52 %, or preferably of 55 % in 28 days.

13. Use of the film forming polyurethane according to any of claims 1 to 11 in cosmetic formulations to increase the biodegradability of the cosmetic formulation.

14. A method for shaping hairstyles or treating hair, styling nails or treating skin in which a film forming polyurethane according to any of claims 1 to 11 or a cosmetic formulation according to claim 12 is applied to hair, nails or skin.

Figure 1

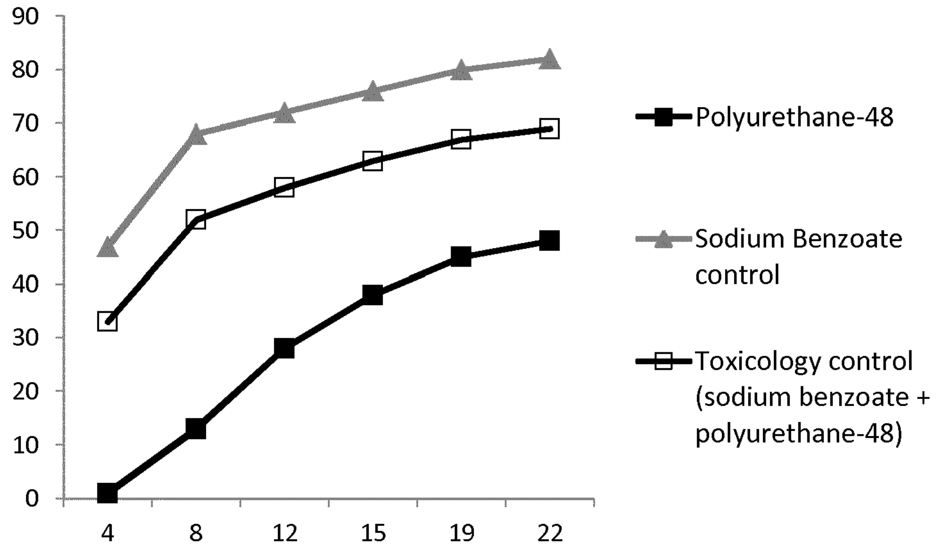


Figure 2

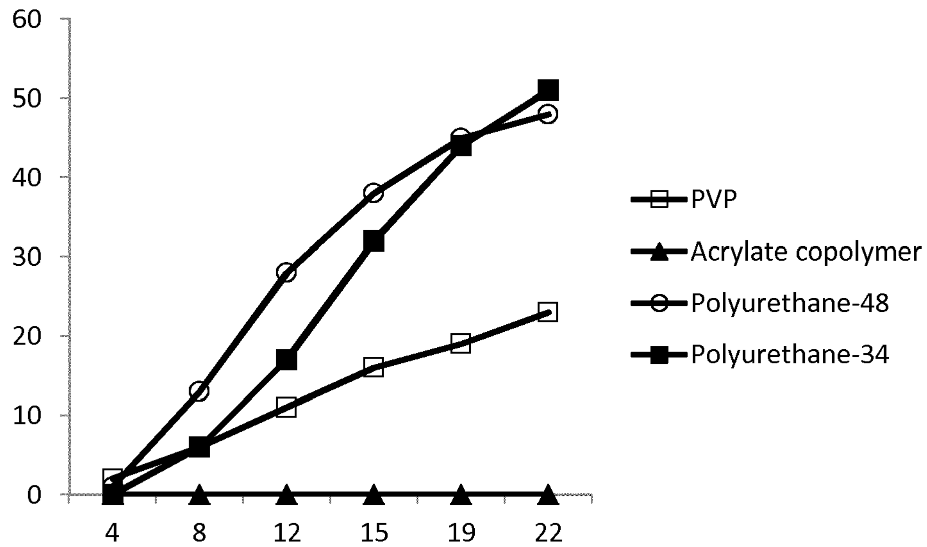

Figure 3

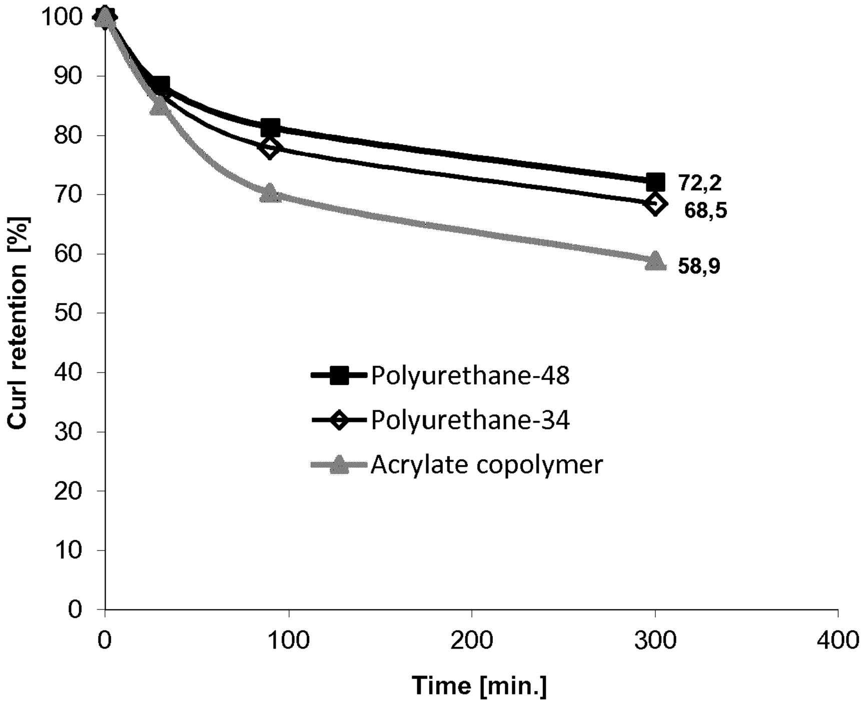


Figure 4

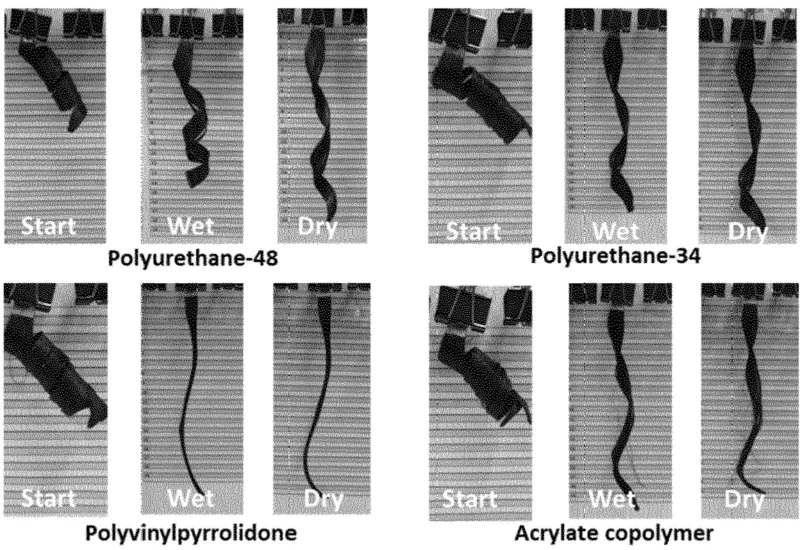

Europäisches Patentamt
European Patent Office
Office européen des brevets

# EUROPEAN SEARCH REPORT

Application Number
EP 18 19 9701

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br>A | WO 2009/118105 A1 (BAYER MATERIALSCIENCE AG [DE]; VIALA SOPHIE [DE]; DOERR SEBASTIAN [DE]) 1 October 2009 (2009-10-01)<br>* pages 35,36,40; examples 3,4 *<br>* page 4, line 17 - page 6, line 16 *<br>----- | 1-12,14<br>13 | INV.<br>C08G18/73<br>A61Q5/06<br>C08G18/75<br>C08G18/08<br>C08G18/42 |
| X<br>A | US 5 961 906 A (MUELLER HANNS-PETER [DE] ET AL) 5 October 1999 (1999-10-05)<br>* column 1, line 46 - column 2, line 62 *<br>* column 5, line 21 - column 6, line 67 *<br>* example 3 *<br>----- | 1-11<br>12-14 | |
| A | EP 1 090 958 A2 (NIPPON CATALYTIC CHEM IND [JP]) 11 April 2001 (2001-04-11)<br>* paragraphs [0012] - [0023], [0042] - [0084]; examples 1-6; tables 1,2 *<br>----- | 1-14 | |

TECHNICAL FIELDS SEARCHED (IPC)

C08G
A61Q

The present search report has been drawn up for all claims

1

| Place of search | Date of completion of the search | Examiner |
|---|---|---|
| Munich | 2 April 2019 | Neugebauer, Ute |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 18 19 9701

This annex lists the patent family members relating to the patent documents cited in the above-mentioned European search report.
The members are as contained in the European Patent Office EDP file on
The European Patent Office is in no way liable for these particulars which are merely given for the purpose of information.

02-04-2019

| Patent document cited in search report | Publication date | Patent family member(s) | Publication date |
|---|---|---|---|
| WO 2009118105 A1 | 01-10-2009 | BR PI0910035 A2 | 29-12-2015 |
| | | CA 2719451 A1 | 01-10-2009 |
| | | CN 101980691 A | 23-02-2011 |
| | | EP 2105127 A1 | 30-09-2009 |
| | | EP 2271304 A1 | 12-01-2011 |
| | | JP 2011515425 A | 19-05-2011 |
| | | KR 20100127792 A | 06-12-2010 |
| | | RU 2010143443 A | 10-05-2012 |
| | | TW 201004655 A | 01-02-2010 |
| | | WO 2009118105 A1 | 01-10-2009 |
| US 5961906 A | 05-10-1999 | AT 188974 T | 15-02-2000 |
| | | DE 19517185 A1 | 14-11-1996 |
| | | EP 0824557 A1 | 25-02-1998 |
| | | ES 2142583 T3 | 16-04-2000 |
| | | JP H11504965 A | 11-05-1999 |
| | | US 5961906 A | 05-10-1999 |
| | | WO 9635733 A1 | 14-11-1996 |
| EP 1090958 A2 | 11-04-2001 | DE 60029289 T2 | 26-07-2007 |
| | | EP 1090958 A2 | 11-04-2001 |
| | | US 6521717 B1 | 18-02-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 2446440 A
- WO 2016116403 A1
- WO 200911813 A1
- WO 2009118105 A1


### Non-patent literature cited in the description


- Ullmanns Encyclopädie der technischen Chemie [Ullmann's Encyclopedia of Industrial Chemistry. Verlag Chemie, vol. 19, 31-38
- Microemulsions Theory and Practice. Academic Press, 1977, 21-32